# FASCICULE DE BREVET EUROPEEN

(11) **EP 3 137 911 B1**
(45) Date de publication et mention de la délivrance du brevet: **11.09.2019**
(21) Numéro de dépôt: 14720962.1
(22) Date de dépôt: 29.04.2014
(51) Int. Cl.: G01N 33/92

(54) **PROCEDE DE DOSAGE D'ACIDES GRAS ERYTHROCYTAIRES**
VERFAHREN ZUR BESTIMMUNG VON FETTSÄUREN IN ERYTHROZYTEN
METHOD FOR ASSAYING FATTY ACIDS IN ERYTHROCYTES

(43) Date de publication de la demande: 08.03.2017
(73) Titulaire: SYNLAB Belgium, 6220 Heppignies (Fleurus) (BE)
(72) Inventeur: VERHOEFT, André, 1390 Grez-doiceau (BE)
(74) Mandataire: Gevers Patents
(86) Numéro de dépôt international: PCT/EP2014/058741
(87) Numéro de publication internationale: WO 2015/165509

(56) Documents cités:
- EP-A1- 2 597 142
- WO-A2-2004/087737
- US-A1- 2009 083 882
- US-A1- 2010 021 956
- GRENON S.M. ET AL.: "Association between n-3 polyunsaturated fatty acid content of red blood cells and inflammatory biomarkers in patients with peripheral artery disease", J. VASC. SURG., vol. 58, no. 5, novembre 2013 (2013-11), pages 1283-1290, XP028749815,
- POTTALA J.V. ET AL.: "Red blood cell fatty acids are associated with depression in a case-control study of adolescents", PROSTAGLANDINS, LEUKOTRIENES AND ESSENTIAL FATTY ACIDS, vol. 86, no. 4, avril 2012 (2012-04), pages 161-165, XP028419501,
- EDER K.: "Gas chromatographic analysis of fatty acid methyl esters", J. CHROMATOGR. B, vol. 671, no. 1, 15 septembre 1995 (1995-09-15), pages 113-131, XP004043942,
- BREKKE H.K. ET AL.: "Lifestyle changes can be achieved through counseling and follow-up in first-degree relatives of patients with type 2 diabetes", J. AM. DIET. ASS., vol. 103, no. 7, juillet 2003 (2003-07), pages 835-843, XP005647688,
- SHANNON J. ET AL.: "Erythrocyte fatty acids and prostate cancer risk: A comparison of methods", PROSTAGLANDINS, LEUKOTRIENES AND ESSENTIAL FATTY ACIDS, vol. 83, no. 3, septembre 2010 (2010-09), pages 161-169, XP027271475,

## Description

La présente invention se rapporte à un procédé de dosage d'acides gras érythrocytaires qui est appliqué dans le cadre d'analyses en biologie nutritionnelle et fonctionnelle.

Les acides gras sont des acides carboxyliques à chaîne aliphatique et possèdent une chaîne carbonée constituée de 4 à 36 atomes de carbone. La biosynthèse des acides gras est réalisée en présence d'acétyl-coenzyme A qui présente deux atomes de carbone. Ainsi, les acides gras contiennent donc toujours un nombre pair d'atomes de carbone. On retrouve les acides gras dans les triglycérides, les esters de cholestérol et les phospholipides.

Chez l'être humain, les acides gras présentent un rôle métabolique car ils constituent une source d'énergie essentielle pour l'organisme. Les acides gras sont contenus sous forme de triglycérides dans les tissus adipeux. Les mécanismes de la β-oxydation permettent la production d'énergie sous forme d'ATP suite à la dégradation par l'organisme des acides gras.

De plus, les acides gras présentent un rôle structural en ce qu'ils contribuent à la synthèse de lipides autres que les triglycérides tels que les phospholipides. Ces derniers forment les membranes situées autour des cellules et des organites. La composition en acides gras des phospholipides dicte les propriétés d'élasticité et de viscosité liées aux membranes.

Les acides gras peuvent également jouer un rôle de messager intra- et extracellulaires. Par exemple, l'acide arachidonique est le précurseur des eicosanoïdes qui sont des hormones qui interviennent en cas d'inflammation.

Les acides gras de la présente invention concernent les acides gras qui sont associés aux phospholipides des membranes érythrocytaires. La concentration des acides gras liés aux phospholipides des membranes érythrocytaires permet de contrôler le métabolisme hépatique, les apports alimentaires, la digestion et l'adsorption desdits acides gras. Une perturbation basée sur la concentration en acides gras déterminée permet d'identifier plusieurs type de pathologies humaines telles que les dépressions, les cancers, les diabètes de type 2, etc.

Les acides gras peuvent appartenir à la famille des acides gras saturés qui présentent des atomes de carbone totalement saturés en hydrogène, des acides gras monoinsaturés dont la chaîne d'atomes de carbone comprend une seule double liaison, des acides gras polyinsaturés dont la chaîne d'atomes de carbone comprend au moins deux doubles liaisons, des acides gras « *trans* » qui sont des acides gras insaturés comprenant au moins une double liaison.

L'acide myristique est un acide gras saturé à 14 atomes de carbone et est produit par le foie à partir de carbohydrate et est obtenu par la consommation de graisses animales telles que la viande bovine, le beurre, la crème fraîche, le fromage, le lard, la noix de coco. La fonction de l'acide myristique consiste à contribuer au fonctionnement des récepteurs aux hormones et au transport de protéines de la cellule vers les mitochondries. Un excès d'acide myristique peut être néfaste pour l'organisme et peut indiquer la présence d'un syndrome métabolique. En effet, il est apparu qu'un excès d'acide myristique active la production de radicaux libres par les polymorphonucléaires neutrophiles (« Myristic Acid, A Side Chain of Phorbol Myristate Acetate (PMA), Can Activate Human Polymorphonuclear Leukocytes to Produce Oxygen Radicals More Potently than PMA» Tada.M and al.; J. Clin. Biochem. Nutr.; 2009 Nov; 45(3):309-14).

Les acides gras « *trans* » sont des acides gras insaturés qui peuvent présenter deux formes géométriques soit les formes « *cis* » et *« trans* ». Chimiquement, les propriétés des deux formes diastéréoisomériques présentent des propriétés différentes. Dans les molécules d'acides gras de type « *trans* », les doubles liaisons entre atomes de carbone sont en conformation « *trans* » et présentent donc une forme relativement droite. La conformation « cis » des acides gras confèrent aux doubles liaisons entre atomes de carbone une forme relativement courbée.

L'acide trans-vaccénique est un exemple d'acide gras de type « *trans* » et provient de la transformation bactérienne d'acides gras insaturés dans le rumen des ruminants. Ces acides gras peuvent donc s'accumuler et se retrouver dans les produits laitiers comme le beurre, la crème ou le lait ; et dans les viandes.

De récentes études ont mis en évidence l'effet bénéfique des acides gras naturels de type trans-vaccénique contre le cancer du sein dans un modèle animal (« Influence of diet enriched with conjugated linoleic acids on their distribution in tissues of rats with DMBA induced tumors » ; Bialek A et al. Lipids Health Dis. 2010 Nov 2 ; 9 : 126). Malgré les résultats de cette récente étude, il est reconnu qu'une quantité trop élevée d'acides gras « *trans* » sont néfastes pour l'organisme.

Les acides gras polyinsaturés de type oméga-6 et oméga-3 sont largement connus dans le domaine de la nutrition. Le dosage desdits acides gras permet de définir un rapport oméga-6/oméga-3 pour identifier un éventuel déséquilibre alimentaire d'un individu, voire un trouble métabolique ou à détecter l'origine d'une pathologie. Le rapport précité représente le rapport entre l'ensemble des acides gras oméga-6 et l'ensemble des acides gras oméga-3. Un rapport préféré serait de 1/1. Le contrôle du rapport oméga-6/oméga-3 permet de mettre en évidence une augmentation éventuelle d'oméga-6. Une telle augmentation peut conduire à des pathologies comme les maladies cardiaques, inflammatoires, auto-immunes et des cancers. Le contrôle dudit rapport est avantageux pour pouvoir prévenir de telles pathologies humaines.

Un exemple de rapport oméga-6/oméga-3 se rapporte au rapport acide arachidonique/acide eicosapentaénoïque qui donne une indication sur le statut pro-normo ou anti-inflammatoire d'un individu en mesurant la proportion entre l'acide arachidonique précurseur des eicosanoïdes qui sont des acides gras pro-inflammatoires et l'acide eicosapentaénoïque qui est un précurseur des eicosanoïdes anti-inflammatoires (« Dietary n-6 and n-3 polyunsaturated fatty acids: from biochemistry to clinical implications in cardiovascular prévention » ; Russo GL., Biochem Pharmacol. 2009 Mar 15;77(6):937-46 et "The importance of the omega-6/omega-3 fatty acid ratio in cardiovascular disease and other chronic diseases"; Simopoulos AP; Exp. Biol. Med. 2008 Jun;233(6):674-88).

Lorsque le rapport acide arachidonique/acide eicosapentaénoïque dépasse une valeur seuil critique, un état pro-inflammatoire peut être observé chez l'individu concerné. Par conséquent, le déclenchement d'un processus anti-inflammatoire dans l'organisme de l'individu sera anormalement renforcé par la production excessive d'éicosanoïdes pro-inflammatoire. L'obtention d'un profil d'acides gras de l'individu permet de mettre en lumière un tel déséquilibre pouvant être lié à une augmentation accrue d'acide arachidonique, à une carence d'acide éicosapentaénoïque ou les deux. Ainsi, l'administration d'un traitement efficace et approprié à un tel déséquilibre peut être adoptée par l'individu souffrant d'une telle pathologie.

Il est également possible de contrôler le rapport entre l'acide linoléique et l'acide dihomogammalinolénique. Ce rapport renseigne de l'activité de la delta-6-désaturase qui est une enzyme hépatique qui assure la transformation des précurseurs de deux familles d'acides gras polyinsaturés à savoir l'acide α-linolénique pour les acides gras de type ω3 et l'acide linoléique pour les acides gras appartenant à la famille des ω6. La delta-6-désaturase constitue l'étape limitante de cette voie métabolique des acides gras.

Lorsque le rapport acide linoléique/acide dihomogammalinolénique est augmenté, il apparaît une déficience de l'activité de la delta-6-désaturase qui peut être néfaste pour la santé de l'individu concerné. En effet, la conversion des précurseurs des acides gras de type ω3 et ω6, soit respectivement l'acide linoléique et l'acide α-linolénique. Cela peut se traduire par une carence en acides gras polyinsaturés. En outre, l'activité de la delta-6-désaturase est affectée en cas d'hyperinsulinisme, de diabète, de stress psychoaffectif, de maladie du foie, d'hypothyroïdie. Les cas précités pouvant se présenter lorsque l'individu consomme une trop grande quantité d'acides gras « *trans* » et qu'il est carencé en magnésium, zinc, vitamine B3 ou B6.

Dans un tel cas de figure, il s'avère nécessaire de pouvoir doser les différents types d'acides gras tels que les acides linoléiques, les acides dihomogammalinoléniques et les acides gras « *trans* » pour pouvoir corriger les carences éventuelles détectées.

Le pourcentage d'acide éicosapentaénoïque et d'acide docosahexanoïque permet de déterminer ce qu'on appelle un « indice oméga-3 » pour l'ensemble des acides gras des phospholipides membranaires des globules rouges. Cet indice constitue un indicateur fiable et efficace pour identifier un risque de mort subite par pathologie cardiovasculaire. La détermination de l'indice oméga-3 permet de contrôler une éventuelle augmentation de la quantité d'acides éicosapentaénoïques et docosahexanoïques.

En somme et au vu de tous les cas de figures précités, il est manifeste que les acides gras érythrocytaires sont des éléments majeurs de la constitution des membranes et sont donc des facteurs déterminants dans l'identification de plusieurs pathologies humaines ou leurs origines. Les acides gras contenus dans lesdites membranes jouent un rôle essentiel dans la structure, dans les différentes fonctions de transmission, dans l'inflammation et la coagulation desdites membranes. Il est recommandé que les différents acides gras soient présents en proportion idéale pour éviter de conduire à un déséquilibre dans l'organisme pouvant être responsable de nombreuses pathologies. La quantité desdits acides gras érythrocytaires dépend des apports alimentaires journaliers ainsi que du fonctionnement des chaines métaboliques concernées.

La détermination de la quantité des acides gras érythrocytaires d'intérêt dans le cadre d'une identification pathologique humaine permet de mieux contrôler les mécanismes fonctionnels pouvant survenir dans l'organisme.

Le dosage des acides gras érythrocytaires est effectué sur un échantillon de sang humain et permet d'établir un profil qui met en lumière des éléments différentiels par rapport à des profils de référence issus d'individus sains. Cette mise en évidence d'écart permet de déterminer la présence d'une pathologie liée à une augmentation de la quantité d'acides gras érythrocytaires ou à un déséquilibre entre différents acides gras érythrocytaires ou encore à une carence d'acides gras érythrocytaires. Ainsi, une approche thérapeutique ciblée peut être appliquée sur l'individu concerné.

L'échantillon de sang comprend toutefois des acides gras érythrocytaires mais également d'autres composants constitutifs du sang comme le plasma sanguin. Une des difficultés de la mise en oeuvre d'un procédé de dosage de ces acides gras érythrocytaires réside dans l'isolation des espèces d'intérêts qui permettraient de déterminer ledit écart différentiel car tout échantillon de sang contient de nombreux constituants qui ne présentent pas d'intérêt pour un tel procédé de dosage, mais qui en outre interfèrent avec les analyses.

Eder K., J. Chromatogr. B, 1995, 671(1): 113-131 est une revue sur les méthodes d'analyse chromatographique en phase gazeuse des esters méthyliques d'acides gras. En particulier, la transestérification acide est mentionnée ainsi que les réactifs communément utilisés qui sont l'acide chlorhydrique, l'acide sulfurique et le trifluorure de bore dans du méthanol. Après trans-estérification, les acides gras estérifiés sont extraits deux fois par ajout d'un mélange de n-hexane et d'eau.

Malheureusement, les techniques actuelles de dosage d'acides gras érythrocytaires ne sont pas encore optimales pour permettre un dosage aisé, rapide, reproductible et fiable d'une grande série d'acides gras érythrocytaires en pathologie humaine.

L'invention a pour but de pallier les inconvénients de l'état de la technique en procurant un procédé de dosage d'une grande série d'acides gras érythrocytaires (jusque 25 différents) qui soit aisément réalisable, fiable et reproductible.

Pour résoudre ce problème, il est prévu suivant l'invention un procédé qui comprend les étapes de :
- amenée d'un volume prédéterminé d'un échantillon de sang humain contenant des acides gras érythrocytaires et des composants constitutifs du sang,
- obtention d'une solution concentrée desdits acides gras érythrocytaires,
- estérification desdits acides gras érythrocytaires pour former une solution contenant des esters méthyliques dérivant desdits acides gras érythrocytaires en présence d'un solvant,
- extraction desdits esters méthyliques desdits acides gras érythrocytaires dudit solvant, ladite étape d'extraction comprenant notamment une étape d'évaporation à condensation refroidie du solvant,
- dosage par chromatographie en phase gazeuse couplée à un détecteur à ionisation de flamme desdits esters méthyliques extraits pour obtenir une mesure de la quantité desdits esters méthyliques,
- comparaison desdites quantités mesurées desdits esters méthyliques avec des valeurs prédéterminées de quantités d'esters méthyliques correspondant à des quantités d'acides gras érythrocytaires pour doser lesdits acides gras érythrocytaires dans ledit volume prédéterminé de l'échantillon de sang.

Le dosage d'acides gras érythrocytaires selon la présente invention est donc réalisé par un traitement d'un volume prédéterminé d'un échantillon de sang humain qui contient des acides gras érythrocytaires et des composants constitutifs du sang. Les acides gras érythrocytaires sont alors séparés desdits composants constitutifs du sang, lesquels ne présentent pas d'intérêt pour le dosage selon l'invention, pour obtenir une solution concentrée desdits acides gras érythrocytaires.

Lorsque les espèces à doser sont séparées des composants constitutifs du sang, elles sont simultanément remises en suspension et estérifiées en présence d'un solvant pour former des esters méthyliques qui dérivent directement desdits acides gras érythrocytaires. Cela permet d'obtenir les esters méthyliques de manière concentrée dans un petit volume de solvant qui pourront être identifiés et quantifiés pour établir un profil devant être analysé. En effet, la volatilité des esters méthyliques est supérieure à celle des acides gras ce qui rend l'analyse en chromatographie en phase gazeuse aisée.

L'extraction des esters méthyliques par un solvant et l'évaporation de celui-ci permettent d'obtenir une solution concentrée en esters méthyliques. Il a été constaté de manière surprenante que l'évaporation par condensation refroidie dudit solvant permet d'extraire rapidement, aisément et efficacement les esters méthyliques sans leur porter atteinte, sans perte conséquente desdits esters pourtant volatils également. De cette façon, une solution concentrée en esters méthyliques peut être obtenue.

Le dosage desdites espèces d'intérêt, soit les esters méthyliques, peut alors avoir lieu par chromatographie en phase gazeuse couplée à un détecteur à ionisation de flamme, ce qui permet d'identifier la nature et de mesurer la quantité desdits esters méthyliques extraits. Lesdites quantités mesurées desdits esters méthyliques sont comparées avec des valeurs prédéterminées de quantités d'esters méthyliques correspondant à des quantités d'acides gras érythrocytaires pour doser lesdits acides gras érythrocytaires dans ledit volume prédéterminé de l'échantillon de sang.

En conséquence, le procédé selon la présente invention permet de traiter l'échantillon de sang avec une méthode de haute qualité, ce qui détermine la fiabilité et la reproductibilité du procédé de dosage utilisé.

Avantageusement, l'étape d'évaporation est réalisée en appliquant un vide de 37 mbar à température ambiante durant 10 minutes à 80 tpm pour piéger le solvant à froid. Cela permet d'encore concentrer la solution obtenue en espèces à doser, sans perte ni dégradation conséquente.

De préférence, ladite étape de dosage par chromatographie en phase gazeuse est réalisée en appliquant une température initiale de 60 °C durant 4 minutes, puis en augmentant successivement la température de 6 °C/minute jusqu'à 120 °C durant 1 minute et de 8 °C/minute jusqu'à 240 °C durant 8,5 minutes pour séparer les esters méthyliques en fonction de leur temps de rétention et une augmentation de 20 °C par minute jusqu'à 250 °C. L'avantage d'un tel programme de température réside dans sa capacité à efficacement séparer les différents acides gras de façon à obtenir un signal qui soit aisément lisible et dont les pics repris sur le chromatogramme soient suffisamment séparés pour identifier la nature et pouvoir mesurer la hauteur des pics de chaque esters méthylique correspondant à un acide gras considéré dans le dosage.

Dans une forme de réalisation particulièrement avantageuse du dispositif selon l'invention, ladite étape d'obtention d'une solution concentrée desdits acides gras érythrocytaires comprend les étapes de :
- mélange dudit volume prédéterminé dudit échantillon de sang avec des anticoagulants,
- centrifugation du mélange jusqu'à la formation de deux phases, la première phase comprenant les composants constitutifs du sang, la deuxième phase contenant les acides gras érythrocytaires,
- séparation desdites deux phases pour isoler lesdits acides gras érythrocytaires desdits composants constitutifs dans une phase liquide, et
- lavage, agitation, centrifugation et isolation de la phase liquide constituée desdits acides gras érythrocytaires jusqu'à l'obtention d'une solution concentrée en acides gras érythrocytaires.

De plus, dans une forme de réalisation particulière, ladite étape d'estérification des acides gras érythrocytaires comprend les étapes de:
- addition à ladite solution concentrée en acides gras érythrocytaires d'un solvant et d'une solution d'estérification contenant de l'acide chlorhydrique et du méthanol pour obtenir un mélange,
- mise à l'étuve dudit mélange durant 2 heures à 95°C,
- agitation dudit mélange toutes les 30 minutes, et
- refroidissement dudit mélange estérifié pour former des esters méthyliques dérivant desdits acides gras érythrocytaires.

Selon un mode préféré, ladite étape d'extraction desdits esters méthyliques desdits acides gras érythrocytaires comprend, avant l'étape d'évaporation, de plus les étapes de :
- addition d'une solution contenant de l'hydrogénocarbonate de sodium saturé à ladite solution dérivée contenant les esters méthyliques,
- addition d'un solvant en présence d'eau, de préférence de l'eau bidistillé,
- agitation et centrifugation de la solution pour former une phase organique,
- récupération de ladite phase organique,
- addition d'un solvant, de préférence du n-hexane, à ladite phase organique, et
- extraction desdits esters méthyliques desdits acides gras érythrocytaires.

L'ajout d'une solution contenant de l'hydrogénocarbonate de sodium saturé à ladite solution dérivée contenant les esters méthyliques permet d'éviter la formation d'une émulsion.

Dans une variante du procédé selon l'invention, lesdits anticoagulants sont choisis dans le groupe constitué d'EDTA, d'héparine, de dérivés fluorés.

De manière particulière avantageuse, ladite étape de lavage est réalisée en présence d'une solution de lavage qui contient un mélange constitué de NaCl dissout dans de l'eau ultra pure caractérisée par une résistivité de 18,2 MΩ.cm à 25°C correspondant à une conductivité de 0,055 µS/cm à 25°C, qui présente une valeur en carbone organique total (COT) inférieure à 5 ppb.

De préférence, ladite solution d'estérification contient de l'acide chlorhydrique concentré, tel que de l'acide chlorhydrique de grade pour analyse, en particulier de l'acide chlorhydrique à 37 %.

De manière encore plus préférentielle, chaque étape de centrifugation est réalisée durant une période de 5 minutes pour une rotation comprise entre 3400 et 4000 tours par minutes (tpm), de préférence de 3600 tpm.

Avantageusement, la durée de chaque étape d'agitation est choisie dans la plage allant de 3 à 300 secondes, de préférences de 20 secondes.

Selon un mode de réalisation préféré, lesdits solvants sont choisis dans le groupe constitué du n-hexane.

D'autres formes de réalisation du dispositif, procédé suivant l'invention sont indiquées dans les revendications annexées.

L'invention se rapport également à une utilisation du procédé de dosage selon l'invention. L'utilisation comprenant les étapes de :
- établissement d'un profil de données à analyser, lesdites données étant obtenues à l'issue du procédé,
- comparaison du profil de données établi avec une série de profils prédéterminés enregistrés dans un espace de stockage de données,
- détermination d'un écart différentiel entre ledit profil établi et lesdits profils prédéterminés et,
- identification d'un déclin cognitif ou de troubles de l'humeur.

De manière avantageuse, le procédé de dosage suivant l'invention est utilisé en biologie nutritionnelle et fonctionnelle.

De préférence, le procédé de dosage selon l'invention est utilisé pour déterminer le fonctionnement de membranes cellulaires et notamment des neurones.

De manière encore plus avantageuse, le procédé de dosage suivant la présente invention est utilisé pour identifier des pathologies cardiovasculaires, des cancers, des dépressions, des inflammations, du diabète de type 2, des maladies rénales chroniques.

D'autres formes de réalisation de l'utilisation suivant l'invention sont indiquées dans les revendications annexées.

D'autres caractéristiques, détails et avantages de l'invention ressortiront de la description donnée ci-après, à titre non limitatif et en faisant référence aux dessins annexés.

Le procédé de dosage suivant l'invention comprend une amenée d'un volume prédéterminé d'un échantillon de sang contenant des acides gras érythrocytaires et des composants constitutifs du sang.

Le volume prédéterminé de l'échantillon de sang peut ensuite être mélangé à un anticoagulant comme de l'EDTA pour former une solution prête à être centrifugée. De cette façon, la phase contenant les espèces d'intérêts est prélevée, isolée desdits composants constitutifs du sang et lavée jusqu'à obtenir une phase concentrée en acides gras érythrocytaires. La phase peut être lavée à l'aide d'une solution de NaCl. La solution concentrée en acides gras érythrocytaires ainsi obtenue est ensuite estérifiée en présence d'un solvant et d'une solution d'estérification, de préférence une solution contenant de l'acide chlorhydrique et du méthanol. La réaction d'estérification permet donc de former des esters méthyliques qui sont des espèces plus volatiles que les acides gras érythrocytaires. Pour cette raison, les esters méthyliques sont préférés car le dosage peut être effectué aisément par chromatographie en phase gazeuse.

Pour que la réaction d'estérification soit efficace et puisse permettre la formation des esters méthyliques, il a été observé que l'application d'un programme de température prédéterminé à permis de séparer correctement et aisément chaque acide gras érythrocytaire. De plus, la réaction d'estérification doit pouvoir être favorable afin de fournir les espèces devant être dosées. La cinétique de la réaction d'estérification est augmentée par chauffage.

Les esters méthyliques doivent de préférence encore être extraits de la solution obtenue après estérification. Pour ce faire, un solvant, de préférence du n-hexane, et de l'eau, de préférence sous forme d'eau bistillée, sont ajoutés à la solution comprenant les esters méthyliques. Une phase organique qui comprend les espèces à doser est obtenue en agitant et centrifugeant la solution précitée. Ainsi, la phase organique est récupérée et mise dans un évaporateur sous vide à température ambiante pour piéger le solvant par condensation refroidie.

Ainsi, les esters méthyliques dérivant des acides gras érythrocytaires peuvent être dosés par chromatographie en phase gazeuse couplée à un détecteur à ionisation de flamme.

Le dosage selon l'invention permet ainsi la séparation des espèces à doser de type esters méthyliques. Un profil comprenant différents pics caractéristiques de chaque espèce est obtenu. Grace au traitement particulier dans le procédé selon la présente invention, chaque espèce à doser présente un temps de rétention qui lui est propre, ce qui permet de ne pas obtenir des pics qui se chevauchent et qui deviennent illisibles lors de l'interprétation des résultats de dosage. L'identification et la quantification de chaque pic sont réalisées en mesurant la surface sous le pic pour déterminer la quantité d'esters méthyliques présents dans l'échantillon initial tandis que l'ionisation à la flamme permet de détecter les esters méthyliques correspondant aux pics du chromatogramme et donc de déterminer l'acide gras érythrocytaire dont il provient.

Le dosage des acides gras érythrocytaires dans le volume prédéterminé de l'échantillon de sang est réalisé en comparant les quantités mesurées des esters méthyliques avec des valeurs prédéterminées de quantités d'esters méthyliques correspondant à des quantités d'acides gras érythrocytaires.

Le procédé de dosage suivant l'invention permet de mesurer les quantités d'acides gras identifiés en pourcentage en poids et peut être utilisé pour mettre en lumière un déséquilibre pouvant conduire à de nombreuses pathologies humaines. L'utilisation du procédé consiste alors à établir un profil de données issu d'un échantillon de sang d'un individu. L'analyse consiste à comparer le profil de l'individu avec une série de profils d'individus qui sont considérés comme étant sain.

Ainsi, la comparaison des profils permet de mettre en évidence, si le cas se présente, un écart différentiel correspondant entre autres à une augmentation du taux d'acides gras érythrocytaires ou à une augmentation d'un rapport d'acides gras érythrocytaires ou encore à une carence en acides gras érythrocytaires. Cela pouvant être un élément de prévention de diverses pathologies humaines en biologie nutritionnelle et fonctionnelle.

### EXEMPLE 1

Un échantillon de sang présentant un volume prédéterminé est centrifugé durant 5 minutes à 3600 tpm en présence d'EDTA et forme ainsi deux phases. La première phase comprend des composants constitutifs du sang et la deuxième phase comprend des acides gras érythrocytaires.

un volume prédéterminé compris entre 0,2 et 1 ml, en particulier entre 0,3 et 0,8 ml, de préférence de 0,4 ml d'érythrocytes est prélevé et transféré dans un tube en verre (16 x 100 mm) auquel sont ajoutés environ successivement 3 volumes d'une solution de NaCl (8766 mg de NaCl par litre d'eau) pour obtenir une solution. Cette dernière est vortexée durant 20 secondes puis centrifugée durant 5 minutes à 3600 tpm. La phase supérieure est extraite. Ainsi cette opération est répétée jusqu'à l'obtention d'une solution concentrée en acides gras érythrocytaires.

La solution concentrée en acides gras érythrocytaires est estérifiée en mélangeant 1 volume, de préférence 40 µl, de la solution concentrée en acides gras érythrocytaires avec 10 volumes, de préférence 400 µl, de n-hexane et 10 volumes ,de préférence 400 µl, d'une solution d'estérification qui comprend de l'acide chlorhydrique à 37 % et du méthanol (par exemple, 12,33 ml d'HCl à 37 % dans un jaugé de 100 ml en présence de méthanol). La solution ainsi obtenue est fermée hermétiquement dans un tube, mélangée et mise à l'étuve durant 2 heures à 95 °C. Le tube est agité toutes les 30 minutes jusqu'à obtenir un mélange estérifié dans lequel les acides gras érythrocytaires ont été transformés en esters méthyliques.

L'extraction des esters méthyliques est réalisée en ajoutant audit mélange estérifié 20 volumes, de préférence 800 µl, d'une solution de NaHCO₃ saturé, et 100 volumes, de préférence 4 ml, d'un mélange de n-hexane et d'eau (75-25), de préférence sous forme d'eau bidistillée. Le mélange ainsi obtenu est vortexé plusieurs fois durant 5 minutes et ensuite centrifugé durant 5 minutes à 4000 tpm à 6 °C. Cela conduit à la formation notamment d'une phase organique qui est récupérée et évaporée à l'aide d'un évaporateur sous vide, par exemple de type Labconco RapidVap (Chrom0125). Cet évaporateur sous vide est muni d'une pompe qui permet de piéger le n-hexane à froid et ainsi permettre le traitement d'un grand nombre d'échantillon (69 tubes) de sang sans atteindre à la qualité du dosage. Les conditions de l'évaporateur sont les suivantes : 80 tpm, température inférieure à 30 °C et 37 mbar.

Lorsque l'évaporation est terminée, l'échantillon est resuspendu dans 0,5 ml de n-hexane et vortexé durant 3 secondes.

Le dosage peut alors avoir lieu et consiste à analyser la solution par chromatographie en phase gazeuse qui va permettre de séparer les esters méthyliques qui présenteront différents temps de rétention dans la colonne. Cette dernière peut être une colonne Supelco SP 2560 (0,18 mm x 75 m x 0,14 µm).

Les conditions de températures de chromatographie consistent à appliquer une température initiale de 60 °C qui est maintenue durant 4 minutes. Ensuite, la température est augmentée de 6°C/minute jusqu'à 120 °C durant 1 minute. Enfin, la température est à nouveau augmentée de 8 °C/minute durant 8,5 minutes. Enfin, la température est augmentée de 20 °C par minutes jusqu'à 250 °C.

Une fois la séparation terminée, l'identification des esters méthyliques est réalisée par détection à ionisation de flamme.

Les résultats obtenus après dosage de l'échantillon d'urine sont repris dans le tableau 1.

**TABLEAU 1**

| La limite de détection est généralement de 0,015 % et la limite de quantification est de 0,03 %. | | |
|---|---|---|
| Formule | Nom | (% en poids) |
| C14 :0 | acide myristique | 0.16 - 0.40 |
| C15 :0 | acide pentadécanoïque | 0.15 - 0.45 |
| C16 :0 | acide palmitique | 21.16 - 23.53 |
| C16:1 9t | acide palmitéléidique | 0.100 - 0.180 |
| C16 :1n7 | acide palmitoléique | 0.180 - 0.490 |
| C17 | acide margarique | 0.04 - 0.06 |
| C18 :0 | acide stéarique | 13.43 - 17.17 |
| C18 :1n7 | acide cis-vaccénique | 0.67 - 0.96 |
| C18 :1n7t | acide trans-vaccénique | 0.04 - 0.19 |
| C18:1n9 | acide oléique | 12.00 - 14.70 |
| C18 :1n9t | acide trans-élaïdique | 0.02 - 0.08 |
| C18 :2n6 | acide linoléique | 8.49 - 11.15 |
| C18 :3n3 | acide α-linolénique | 0.08 - 0.17 |
| C18 :3n6 | acide γ-linolénique | 0.04 - 0.09 |
| C20 :1n9 | acide gadoléique | 0.15 - 0.21 |
| C18:2 9c 11t | acide ruménique | 0.07 - 0.15 |
| C20 :3n6 | acide dihomogammalinolénique | 1.28 - 2.20 |
| C20 :4n6 | acide arachidonique | 11.00 - 13.44 |
| C24:0 | acide lignocérique | 5.18 - 6.06 |
| C20 :5n3 | acide éicosapentaénoïque | 0.75 - 2.34 |
| C24:1 15c | acide nervonique | 5.00 - 6.54 |
| C22:4 | acide adrénique | 1.84 - 3.72 |
| C22 :5n6 | acide docosapentaénoïque | 0.17 - 0.37 |
| C22 :6n3 | acide docosahexaénoïque | 5.59 - 6.40 |

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé de dosage d'acides gras érythrocytaires, comprenant les étapes de :
- amenée d'un volume prédéterminé d'un échantillon de sang humain contenant des acides gras érythrocytaires et des composants constitutifs du sang,
- obtention d'une solution concentrée desdits acides gras érythrocytaires,
- estérification desdits acides gras érythrocytaires pour former une solution contenant des esters méthyliques dérivant desdits acides gras érythrocytaires en présence d'un solvant,
- extraction desdits esters méthyliques desdits acides gras érythrocytaires dudit solvant, ladite étape d'extraction comprenant une étape d'évaporation à condensation refroidie du solvant,
- dosage par chromatographie en phase gazeuse couplée à un détecteur à ionisation de flamme desdits esters méthyliques extraits pour obtenir une mesure de la quantité desdits esters méthyliques,
- comparaison desdites quantités mesurées desdits esters méthyliques avec des valeurs prédéterminées de quantités d'esters méthyliques correspondant à des quantités d'acides gras érythrocytaires pour doser lesdits acides gras érythrocytaires dans ledit volume prédéterminé de l'échantillon de sang.

2. Procédé selon la revendication 1, dans lequel l'étape d'évaporation est réalisée en appliquant un vide de 37 mbar à température ambiante durant 10 minutes à 80 tpm pour piéger le solvant à froid.

3. Procédé selon la revendication 1 ou la revendication 2, dans lequel ladite étape de dosage par chromatographie en phase gazeuse est réalisée en appliquant une température initiale de 60 °C durant 4 minutes, puis en augmentant successivement la température de 6 °C/minutes jusqu'à 120 °C durant 1 minutes et de 8 °C/minutes jusqu'à 240 °C durant 8,5 minutes pour séparer les esters méthyliques en fonction de leur temps de rétention et une augmentation de 20 °C par minute jusqu'à 250 °C.

4. Procédé selon la revendication 1, dans lequel ladite étape d'obtention d'une solution concentrée desdits acides gras érythrocytaires comprend les étapes de :
- mélange dudit volume prédéterminé dudit échantillon de sang avec des anticoagulants,
- centrifugation du mélange jusqu'à la formation de deux phases, la première phase comprenant les composants constitutifs du sang, la deuxième phase contenant les acides gras érythrocytaires,
- séparation desdites deux phases pour isoler lesdits acides gras érythrocytaires desdits composants constitutifs dans une phase liquide, et
- lavage, agitation, centrifugation et isolation de la phase liquide constituée desdits acides gras érythrocytaire jusqu'à l'obtention d'une solution concentrée en acides gras érythrocytaires.

5. Procédé selon la revendication 1, dans lequel ladite étape d'estérification des acides gras érythrocytaires comprend les étapes de :
- addition à ladite solution concentrée en acides gras érythrocytaires d'un solvant et d'une solution d'estérification contenant de l'acide chlorhydrique et du méthanol pour obtenir un mélange,
- mise à l'étuve dudit mélange durant 2 heures à 95°C,
- agitation dudit mélange toutes les 30 minutes, et
- refroidissement dudit mélange estérifié pour former des esters méthyliques dérivant desdits acides gras érythrocytaires.

6. Procédé selon l'une quelconque des revendications précédentes, dans lequel ladite étape d'extraction desdits esters méthyliques desdits acides gras érythrocytaires comprend, avant l'étape d'évaporation, de plus les étapes de :
- addition d'une solution contenant de l'hydrogénocarbonate de sodium saturé à ladite solution dérivée contenant les esters méthyliques,
- addition d'un solvant en présence d'eau, de préférence de l'eau bidistillé,
- agitation et centrifugation de la solution pour former une phase organique,
- récupération de ladite phase organique,
- addition d'un solvant, de préférence du n-hexane, à ladite phase organique, et
- extraction desdits esters méthyliques desdits acides gras érythrocytaires.

7. Procédé selon la revendication 4, dans lequel les anticoagulants sont choisis dans le groupe constitué d'EDTA, d'héparine, de dérivés fluorés.

8. Procédé selon la revendication 4 ou 7, dans lequel ladite étape de lavage est réalisée en présence d'une solution de lavage qui contient un mélange constitué de NaCl dissout dans de l'eau ultra pure **caractérisé par** une résistivité de 18,2 MΩ.cm à 25°C correspondant à une conductivité de 0,055 µS/cm à 25°C, qui présente une valeur en carbone organique total (COT) inférieure à 5 ppb.

9. Procédé selon la revendication 5, dans lequel la solution d'estérification contient de l'acide chlorhydrique concentré, en particulier de grade pour analyse, plus particulièrement de l'acide chlorhydrique à 37 %.

10. Procédé selon l'une quelconque des revendications précédentes, dans lequel chaque étape de centrifugation est réalisée durant une période de 5 minutes pour une rotation comprise entre 3400 et 4000 tpm, de préférence 3600 tpm.

11. Procédé selon l'une quelconque des revendications précédentes, dans lequel la durée de chaque étape d'agitation est choisie dans la plage allant de 3 à 300 secondes, de préférence 20 secondes.

12. Procédé selon l'une quelconque des revendications précédentes, dans lequel lesdits solvants sont choisis dans le groupe constitué du n-hexane.

13. Utilisation du procédé de dosage selon les revendications 1 à 12 comprenant les étapes de :
- établissement d'un profil de données à analyser, lesdites données étant obtenues à l'issue du procédé de dosage selon les revendications 1 à 12,
- comparaison du profil de données établi avec une série de profils prédéterminés enregistrés dans un espace de stockage de données,
- détermination d'un écart différentiel entre ledit profil établi et lesdits profils prédéterminés et,
- identification d'un déclin cognitif ou de troubles de l'humeur.

14. Utilisation du procédé de dosage selon les revendications 1 à 12, en biologie nutritionnelle et fonctionnelle.

15. Utilisation du procédé de dosage selon les revendications 1 à 12, pour déterminer le fonctionnement des membranes cellulaires et notamment des neurones.

16. Utilisation du procédé de dosage selon les revendications 1 à 12, pour identifier des pathologies cardiovasculaires, des cancers, des dépressions, des inflammations, du diabète de type 2, des maladies rénales chroniques.

## Patentansprüche

1. Verfahren zur Gehaltsbestimmung von Erythrozyten-Fettsäuren, die Schritte umfassend:
- Zuführung eines vorbestimmten Volumens einer Probe menschlichen Bluts, die Erythrozyten-Fettsäuren und Blutbestandteile enthält,
- Erhalt einer konzentrierten Lösung der Erythrozyten-Fettsäuren ,
- Veresterung der Erythrozyten-Fettsäuren, um eine Lösung zu bilden, die Methylester enthält, die sich aus den Erythrozyten-Fettsäuren ableiten, bei Vorhandensein eines Lösungsmittels,
- Extraktion der Methylester der Erythrozyten-Fettsäuren aus dem Lösungsmittel, wobei der Extraktionsschritt einen Kaltkondensation-Verdampfungsschritt des Lösungsmittels umfasst,
- Gehaltsbestimmung durch Gaschromatographie gekoppelt mit einem Flammenionisationsdetektor der extrahierten Methylester, um eine Messung der Menge der Methylester zu erhalten,
- Vergleich der gemessenen Mengen der Methylester mit vorbestimmten Mengenwerten an Methylestern, die Mengen von Erythrozyten-Fettsäuren entsprechen, um die Erythrozyten-Fettsäuren im vorbestimmten Volumen der Blutprobe zu dosieren.

2. Verfahren nach Anspruch 1, wobei der Verdampfungsschritt realisiert wird, indem 10 Minuten lang ein Vakuum von 37 mbar bei Raumtemperatur bei 80 UpM angewandt wird, um das Lösungsmittel kalt abzuscheiden.

3. Verfahren nach Anspruch 1 oder Anspruch 2, wobei der Gehaltsbestimmungsschritt durch Gaschromatographie realisiert wird, indem 4 Minuten lang eine Anfangstemperatur von 60 °C angewandt wird, dann indem 1 Minute lang die Temperatur sukzessiv um 6 °C/Minute bis 120 °C und 8,5 Minuten lang um 8 °C/Minute bis 240 °C erhöht wird, um die Methylester in Abhängigkeit von ihrer Retentionszeit zu trennen, und eine Erhöhung um 20 °C pro Minute bis 250 °C.

4. Verfahren nach Anspruch 1, wobei der Schritt des Erhaltes einer konzentrierten Lösung der Erythrozyten-Fettsäuren die Schritte umfasst:
- Mischung des vorbestimmten Volumens der Blutprobe mit Antikoagulanzien,
- Zentrifugierung des Gemisches bis zur Bildung von zwei Phasen, wobei die erste Phase die Blutbestandteile umfasst, die zweite Phase die Erythrozyten-Fettsäuren enthält,
- Trennung der zwei Phasen, um die Erythrozyten-Fettsäuren von den Bestandteilen in einer flüssigen Phase zu isolieren, und
- Spülung, Agitation, Zentrifugierung und Isolierung der aus den Erythrozyten-Fettsäuren bestehenden flüssigen Phase bis zum Erhalt einer an Erythrozyten-Fettsäuren konzentrierten Lösung.

5. Verfahren nach Anspruch 1, wobei der Schritt der Veresterung der Erythrozyten-Fettsäuren die Schritte umfasst:
- Zugabe zur an Erythrozyten-Fettsäuren konzentrierten Lösung eines Lösungsmittels und einer Veresterungslösung, die Chlorwasserstoffsäure und Methanol enthält, um ein Gemisch zu erhalten,
- Platzieren des Gemisches in den Trockenofen, 2 Stunden lang bei 95 °C,
- Agitation des Gemisches alle 30 Minuten, und
- Abkühlen des veresterten Gemisches, um Methylester zu bilden, die sich aus den Erythrozyten-Fettsäuren ableiten.

6. Verfahren nach einem der vorstehenden Ansprüche, wobei der Schritt der Extraktion der Methylester der Erythrozyten-Fettsäuren vor dem Verdampfungsschritt zusätzlich die Schritte umfasst:
- Zugabe einer Lösung, die gesättigtes Natriumhydrogencarbonat enthält, zur abgeleiteten Lösung, die die Methylester enthält,
- Zugabe eines Lösungsmittels bei Vorhandensein von Wasser, vorzugsweise bidestilliertem Wasser,
- Agitation und Zentrifugierung der Lösung, um eine organische Phase zu bilden,
- Rückgewinnung der organischen Phase,
- Zugabe eines Lösungsmittels, vorzugweise n-Hexan, zur organischen Phase, und
- Extraktion der Methylester aus den Erythrozyten-Fettsäuren.

7. Verfahren nach Anspruch 4, wobei die Antikoagulanzien ausgewählt sind aus der Gruppe bestehend aus EDTA, Heparin, fluorierten Derivaten.

8. Verfahren nach Anspruch 4 oder 7, wobei der Schritt der Spülung bei Vorhandensein einer Spülungslösung realisiert wird, die ein Gemisch enthält, das aus in ultrareinem Wasser gelöstem NaCl besteht, **gekennzeichnet durch** einen Widerstand von 18,2 MΩ.cm bei 25 °C entsprechend einer Leitfähigkeit von 0,055 µS/cm bei 25 °C, was einen Wert an gesamtem organischem Kohlenstoff (TOC) von weniger als 5 ppb darstellt.

9. Verfahren nach Anspruch 5, wobei die Veresterungslösung konzentrierte Chlorwasserstoffsäure enthält, insbesondere der Stufe zur Analyse, besonders bestimmt Chlorwasserstoffsäure zu 37 %.

10. Verfahren nach einem der vorstehenden Ansprüche, wobei jeder Zentrifugierungsschritt während einer Dauer von 5 Minuten bei einer Drehung zwischen 3400 und 4000 UpM, vorzugsweise 3600 UpM, realisiert wird.

11. Verfahren nach einem der vorstehenden Ansprüche, wobei die Dauer jedes Agitationsschritts ausgewählt wird aus dem Bereich von 3 bis 300 Sekunden, vorzugsweise 20 Sekunden.

12. Verfahren nach einem der vorstehenden Ansprüche, wobei die Lösungsmittel ausgewählt sind aus der Gruppe bestehend aus n-Hexan.

13. Verwendung des Verfahrens zur Gehaltsbestimmung nach einem der Ansprüche 1 bis 12, umfassend die Schritte:
- Erstellen eines zu analysierenden Datenprofils, wobei die Daten bei Abschluss des Verfahrens zur Gehaltsbestimmung nach einem der Ansprüche 1 bis 12 erhalten werden,
- Vergleich des erstellten Datenprofils mit einer Reihe von in einem Datenspeicherraum gespeicherten vorbestimmten Profilen,
- Ermitteln einer differenziellen Abweichung zwischen dem erstellten Profil und den vorbestimmten Profilen, und
- Identifizierung eines kognitiven Abbaus oder von Gemütsstörungen.

14. Verwendung des Verfahrens zur Gehaltsbestimmung nach einem der Ansprüche 1 bis 12 in der Ernährungs- und funktionellen Biologie.

15. Verwendung des Verfahrens zur Gehaltsbestimmung nach einem der Ansprüche 1 bis 12, um die Tätigkeit von Zellmembranen und insbesondere von Neuronen zu ermitteln.

16. Verwendung des Verfahrens zur Gehaltsbestimmung nach einem der Ansprüche 1 bis 12, um kardiovaskuläre Pathologien, Krebserkrankungen, Depressionen, Entzündungen, Typ-2-Diabetes, chronische Nierenerkrankungen zu identifizieren.

## Claims

1. Method for assaying erythrocyte fatty acids, comprising the steps of:
- supplying a predetermined volume of a human blood sample containing erythrocyte fatty acids and components constituents of blood,
- obtaining a concentrated solution of said erythrocyte fatty acids,
- esterifying said erythrocyte fatty acids to form a solution containing methyl esters derived from said erythrocyte fatty acids in the presence of a solvent,
- extracting said methyl esters of said erythrocyte fatty acids from said solvent, said extracting step comprising a cooled condensation evaporating step of the solvent,
- assaying by gas chromatography coupled with a flame ionization detector of said extracted methyl esters to obtain a measurement of the quantity of said methyl esters,
- comparing said measured quantities of said methyl esters with predetermined values of quantities of methyl esters corresponding to quantities of erythrocytes fatty acids in order to dose said erythrocytes fatty acids in said predetermined volume of the blood sample.

2. Method according to claim 1, wherein the evaporating step is performed by applying a vacuum of 37 mbar at room temperature for 10 minutes at 80 rpm to cold trap the solvent.

3. Method according to claim 1 or claim 2, wherein said gas chromatographic assaying step is performed by applying an initial temperature of 60°C for 4 minutes, then successively increasing the temperature of 6°C/minute to 120°C for 1 minute and of 8°C/minute to 240°C for 8.5 minutes to separate methyl esters according to their retention time and an increase of 20°C per minute to 250°C.

4. Method according to claim 1, wherein said obtaining step of a concentrated solution of said erythrocyte fatty acids comprises the steps of:
- mixing said predetermined volume of said blood sample with anticoagulants,
- centrifuging the mixture until two phases are formed, the first phase comprising the components constituents of blood, the second phase containing the erythrocyte fatty acids,
- separating said two phases to isolate said erythrocytes fatty acids from said components constituents in a liquid phase, and
- washing, stirring, centrifuging and isolating the liquid phase consisting of said erythrocyte fatty acids until obtaining a concentrated solution of erythrocyte fatty acids.

5. Method according to claim 1, wherein said esterifying step of erythrocyte fatty acids comprises the steps of:
- adding a solvent and an esterification solution containing hydrochloric acid and methanol to said concentrated solution of erythrocytes fatty acids to obtain a mixture,
- setting said mixture in the oven for 2 hours at 95°C,
- stirring said mixture every 30 minutes, and
- cooling said esterified mixture to form methyl esters derived from said erythrocyte fatty acids.

6. Method according to any of the preceding claims, wherein said extracting step of said methyl esters from said erythrocytes fatty acids further comprises, prior to the evaporating step, the steps of:
- adding a solution containing saturated sodium hydrogen carbonate to said derived solution containing the methyl esters,
- adding a solvent in the presence of water, preferably bi-distilled water,
- stirring and centrifuging the solution to form an organic phase,
- recovering said organic phase,
- adding a solvent, preferably n-hexane, to said organic phase, and
- extracting said methyl esters from said erythrocytes fatty acids.

7. Method according to claim 4, wherein the anticoagulants are selected from the group consisting of EDTA, heparin, fluorinated derivatives.

8. Method according to claim 4 or 7, wherein said washing step is performed in the presence of a washing solution which contains a mixture constituted of NaCl dissolved in ultra-pure water **characterized by** a resistivity of 18.2 MΩ.cm at 25°C corresponding to a conductivity of 0.055 µS/cm at 25°C, which has a total organic carbon (TOC) value lower than 5 ppb.

9. Method according to claim 5, wherein the esterifying solution contains concentrated hydrochloric acid, in particular of grade for analysis, more particularly hydrochloric acid at 37%.

10. Method according to any of the preceding claims, wherein each centrifuging step is performed over a period of 5 minutes for a rotation between 3400 and 4000 rpm, preferably 3600 rpm.

11. Method according to any of the preceding claims, wherein the duration of each stirring step is selected in the range of 3 to 300 seconds, preferably 20 seconds.

12. Method according to any of the preceding claims, wherein said solvents are selected from the group consisting of n-hexane.

13. Use of the method for assaying according to claims 1 to 12 comprising the steps of:
- establishing a profile of data to be analyzed, said data being obtained from the method for assaying according to claims 1 to 12,
- comparing the established data profile with a series of predetermined profiles stored in a data storage space,
- determining a differential difference between said established profile and said predetermined profiles and,
- identifying a cognitive decline or mood disorders.

14. Use of the method for assaying according to claims 1 to 12 in nutritional and functional biology.

15. Use of the method for assaying according to claims 1 to 12 for determining the functioning of cell membranes and in particular neurons.

16. Use of the method for assaying according to claims 1 to 12, for identifying cardiovascular pathologies, cancers, depressions, inflammations, type 2 diabetes, chronic kidney diseases.
